# FASCICULE DE BREVET EUROPEEN

(11) **EP 1 166 770 B1**
(45) Date de publication et mention de la délivrance du brevet: **04.09.2002**
(21) Numéro de dépôt: 01401576.2
(22) Date de dépôt: 15.06.2001
(51) Int. Cl.: A61K 7/48, A61K 47/36

(54) **Composition cosmétique ou pharmaceutique comprenant un ester de dextrine et un gélifant hydrophile**
Kosmetische oder pharmazeutische Zusammensetzung enthaltend ein Ester des Dextrins und ein hydrophiles Geliermittel
Cosmetic or pharmaceutical composition comprising a dextrin ester and a hydrophilic gelifier

(30) Priorité: 26.06.2000 FR 0008157
(43) Date de publication de la demande: 02.01.2002
(73) Titulaire: L'OREAL, 75008 Paris (FR)
(72) Inventeur: Lorant, Raluca, 94320 Thiais (FR)
(74) Mandataire: Rasson, Catherine

(56) Documents cités:
- EP-A- 0 736 545
- EP-A- 1 066 827
- FR-A- 2 782 269
- FR-A- 2 795 083
- US-A- 4 540 510
- DATABASE CHEMABS [en ligne] CHEMICAL ABSTRACTS SERVICE, COLUMBUS, OHIO, US; Database accession no. 1999:78451 XP002166615 & JP 11 029432 A (SHISEIDO) 2 février 1999 (1999-02-02)
- DATABASE CHEMABS [en ligne] CHEMICAL ABSTRACTS SERVICE, COLUMBUS, OHIO, US; Database accession no. 1994:14642 XP002166616 & JP 05 246824 A (SHISEIDO) 24 septembre 1993 (1993-09-24)
- DATABASE WPI Derwent Publications Ltd., London, GB; AN 1996035826 XP002166619 & JP 07 304669 A (TAISHO PHARM), 21 novembre 1995 (1995-11-21)
- DATABASE CHEMABS [en ligne] CHEMICAL ABSTRACTS SERVICE, COLUMBUS, OHIO, US; Database accession no. 1999:420854 XP002166617 & JP 11 180830 A (SHISEIDO) 6 juillet 1999 (1999-07-06)
- DATABASE CHEMABS [en ligne] CHEMICAL ABSTRACTS SERVICE, COLUMBUS, OHIO, US; Database accession no. 1988:576334 XP002166618 & JP 63 159489 A (SHINETSU CHEMICAL INDUSTRY) 2 juillet 1988 (1988-07-02)

## Description

La présente invention a trait à une composition notamment cosmétique ou pharmaceutique pouvant en particulier se présenter sous forme d'une émulsion, et susceptible d'être utilisée pour le soin et/ou le maquillage de la peau, des semi-muqueuses, des muqueuses et/ou des phanères.

Les compostions cosmétiques, et notamment les composition de soin ou de maquillage de type fond de teint, se présentent généralement sous la forme d'émulsion eau-dans-huile ou huile-dans-eau. Ces émulsions comprennent généralement une phase grasse, une phase aqueuse et au moins un émulsifiant qui apporte de la stabilité à l'émulsion.

Par les émulsifiants susceptibles d'être employés, on peut citer les esters de dextrine et d'acides gras en C10-C24. Ceci est notamment décrit dans JP8/283303 de Chiba Seifun Co qui est relatif à l'utilisation de ces esters de dextrine pour préparer des émulsions eau-dans-huile.

Toutefois, on a constaté que les émulsions ainsi préparées n'étaient pas très stables; en effet, on observe un déphasage rapide de l'émulsion dès que l'on arrête l'agitation.

Il subsiste donc le besoin d'émulsions stables comprenant des esters de dextrine, notamment parce que ces esters sont particulièrement intéressants : en effet, ils présentent l'avantage d'être bien tolérés par la peau et des former des émulsions ayant des propriétés sensorielles agréables.

Or la demanderesse a mis en évidence que l'ajout d'un composé spécifique, à savoir un type de gélifiant hydrophile particulier, dans ces émulsion pouvait permettre d'améliorer de façon remarquable la stabilité desdites émulsions E/H.

Par ailleurs, on a également constaté avec surprise que l'utilisation de l'association desdits esters de dextrine et dudit gélifiant hydrophile pouvait également permettre la préparation d'émulsions H/E alors que les esters de dextrine ne sont connus que comme émulsionnant E/H. Les émulsions H/E ainsi obtenues sont également particulièrement stables, y compris lorsque la quantité d'huile est importante, par exemple de l'ordre de 30-40% en poids. En outre, elles ont de bonnes propriétés cosmétiques (compositions lisses), ce qui n'est pas le cas par exemple des compositions contenant un ester de dextrine et un polymère carboxyvinylique de type carbomer, qui sont granuleuses et moins onctueuses.

La présente invention a donc pour but de proposer une nouvelle association permettant la préparation d'émulsions stables, qui soient aussi bien de type E/H que de type H/E, voire multiple.

La présente invention a pour objet une composition notamment cosmétique ou pharmaceutique comprenant, dans un milieu cosmétiquement ou pharmaceutiquement acceptable, au moins un ester de dextrine et d'acide gras répondant à la formule (I) définie ci-après et au moins un gélifiant hydrophile de type polymérique choisi parmi les polymères de type acrylamide ou dérivés et les copolymères constitués d'une fraction majoritaire de monomère acide carboxylique monooléfiniquement insaturé en C₃-C₆ ou de son anhydride, et d'une fraction minoritaire de monomère ester à chaîne grasse d'acide acrylique.

Les compositions selon l'invention sont facilement applicables sur la peau et permettent entre autre d'obtenir des produits cosmétiques, notamment de soin ou de maquillage, ayant une texture agréable, lisse, peu collante, qui restent confortables à porter tout au long de la journée.

De plus, leurs propriétés cosmétiques sont très intéressantes : elles procurent de la fraîcheur à l'application, un effet nutritif et apaisant dès l'application, ainsi qu'une grande douceur en final.

Les émulsions E/H sont stables sans utilisation d'autres émulsionnants et/ou d'agents de consistance; l'utilisation d'un gélifiant hydrophile en association avec un ester de dextrine permet d'obtenir des émulsions E/H plus légères, plus fraîches et plus agréables à porter que lorsque l'ester de dextrine est employé seul.

Les émulsions H/E sont crémeuses et onctueuses; elles sont fraîches et légères. Par ailleurs, lorsque le taux de phase huileuse est important, par exemple d'au moins 20% en poids, les émulsions obtenues sont riches, nutritives, apaisantes et bien adaptées au soin des peaux sèches et sensibles.

D'une manière générale, les émulsions selon l'invention sont stables sans nécessiter de quantité importante d'émulsionnant, d'où un avantage en terme d'innocuité, de douceur sur la peau et de confort/agrément cosmétique. Cette bonne stabilité est vérifiée même en présence d'un fort taux de phase interne.

Les compositions selon l'invention trouvent notamment une application particulièrement intéressante dans le domaine du soin et/ou du maquillage de la peau, des muqueuses, des semi-muqueuses, et des phanères. On entend notamment par muqueuse, la partie interne de la paupière inférieure; parmi les semi-muqueuses, on entend plus particulièrement les lèvres du visage; par phanères, on entend les cils, sourcils, cheveux et ongles. Ainsi, l'invention trouve une application toute particulière dans le domaine des produits de soin, de nettoyage, de démaquillage et/ou de maquillage des lèvres du visage et de la peau, tels que les fonds de teint, les autobronzants, les produits solaires et les produits de soin, de nettoyage ou de démaquillage de la peau.

La composition selon l'invention comprend donc au moins un ester de dextrine et d'acide gras, répondant à la formule (I) : 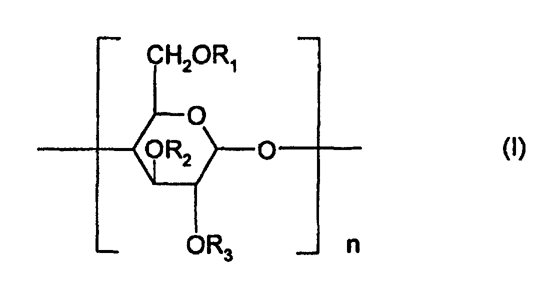 dans laquelle :
- les radicaux R₁, R₂ et R₃, identiques ou différents, sont choisis parmi l'hydrogène ou un groupement acyle (R-CO-) dans lequel le radical R est un groupement hydrocarboné, linéaire ou ramifié, saturé ou insaturé, possédant 6 à 30, notamment 8 à 22, voire 12-18, atomes de carbone, sous réserve qu'au moins un desdits radicaux R₁, R₂ ou R₃ est différent de l'hydrogène,
- n est un entier compris entre 3 et 150, notamment 10 et 100, et de préférence 15-40.

De préférence, les trois radicaux R1, R2 et R3 sont différents de l'hydrogène, ce qui conduit à des esters de dextrine ayant la formule (II) suivante : 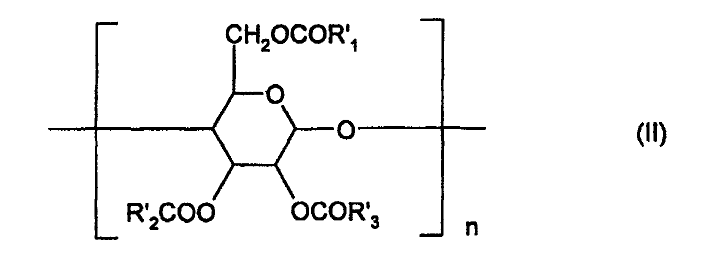 dans laquelle les radicaux R'₁, R'₂ et R'₃, identiques ou différents, représentent un groupement hydrocarboné, linéaire ou ramifié, saturé ou insaturé, possédant 6 à 30, notamment 8 à 22, plus particulièrement 12-18 atomes de carbone, - n est un entier compris entre 3 et 150, notamment 10 et 100, et de préférence 15-40.

Notamment les radicaux -OCOR'₁, -OCOR'₂ et/ou -OCOR'₃ peuvent être choisis parmi les radicaux caprylique, caprique, laurique, myristique, palmitique, stéarique, arachique, behenique, isobutyrique, isovalérique, éthyl-2 butyrique, éthylméthylacétique, isoheptanoïque, éthyl-2 hexanoïque, isononanoïque, isodécanoïque, isotridécanoïque, isomyristique, isopalmitique, isostéarique, isoaracique, isohexanoïque, decenoïque, dodécenoïque, tetradecenoïque, myristoléïque, hexadécénoïque, palmitoléïque, oléïque, élaidique, asclepinique, gondoléïque, eicosènoïque, sorbique, linoléïque, linolénique, punicique, stéaridonique, arachidonique, stéarolique, et leurs mélanges.

De préférence, on utilise un palmitate de dextrine.

Certains de ces esters de dextrine sont disponibles commercialement, notamment sous la dénomination RHEOPEARL de la société Chiba Seifun Co.

Ces esters de dextrine peuvent être présents dans la composition selon l'invention en une quantité de 0,2-10%, de préférence de 0,5-5% en poids, et plus préférentiellement de 1-4% en poids, par rapport au poids total de la composition.

La composition selon l'invention comprend par ailleurs au moins un gélifiant hydrophile de type polymérique, choisi parmi les polymères de type acrylamide ou dérivés, parmi les copolymères constitués d'une fraction majoritaire de monomère acide carboxylique monooléfiniquement insaturé en C₃-C₆ ou de son anhydride, et d'une fraction minoritaire de monomère ester à chaîne grasse d'acide acrylique, et leurs mélanges.

Le gélifiant hydrophile choisi parmi les polymères de type acrylamide ou dérivés peut être notamment choisi parmi les polymères poly(acide 2-acrylamido 2-méthylpropane sulfonique) éventuellement réticulés et neutralisés et les copolymères anioniques réticulés d'acrylamide et d'acide 2-acrylamido 2-méthylpropane sulfonique.

Selon un mode préféré de l'invention, ce gélifiant hydrophile polymérique est notamment un polymère poly(acide 2-acrylamido 2-méthylpropane sulfonique) réticulé et neutralisé à au moins 90%.

Ces polymères permettent notamment d'obtenir des propriétés cosmétiquement agréables, telles que de la douceur et de la facilité d'étalement, avec une gamme de viscosité des compositions finales importantes, pouvant aller de la forme d'un lait fluide à la forme d'une crème. En outre, les compositions obtenues sont lisses et agréables à utiliser.

Ce polymère poly(acide 2-acrylamido 2-méthylpropane sulfonique) réticulé et pratiquement ou totalement neutralisé est généralement hydrosoluble ou gonflable dans l'eau. Il peut être caractérisé par le fait qu'il comprend, distribués de façon aléatoire :
a) de 90 à 99,9% en poids de motifs de formule générale (III) suivante : 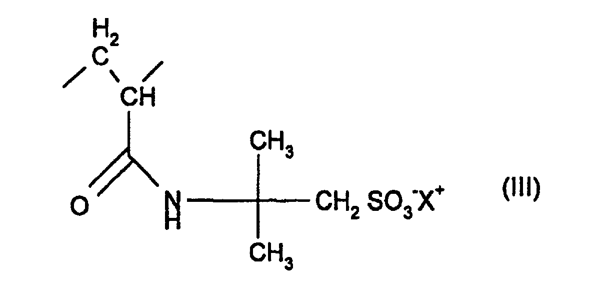 dans laquelle X⁺ désigne un cation ou un mélange de cations, au plus 10% molaire des cations X⁺ pouvant être des protons H⁺ ; et
b) de 0,01 à 10% en poids de motifs réticulants provenant d'au moins un monomère ayant au moins deux doubles liaisons oléfiniques ; les proportions en poids étant définies par rapport au poids total du polymère.

Ce polymère comprend de préférence de 98 à 99,5 % en poids de motifs de formule (1) et de 0,2 à 2 % en poids de motifs réticulants.

Le cation X⁺ représente un cation ou un mélange de cations choisis en particulier parmi un proton, un cation de métal alcalin, un cation équivalent de celui d'un métal alcalino-terreux ou l'ion ammonium. Le cation X⁺ préférentiel est le cation NH₄⁺. Plus particulièrement, 90 à 100% mole des cations sont des cations NH₄⁺ et 0 à 10% mole sont des protons (H⁺).

Les monomères de réticulation ayant au moins deux doubles liaisons oléfiniques sont choisis par exemple parmi le dipropylèneglycol-diallyléther, les polyglycol-diallyléthers, le triéthylèneglycol-divinyléther, l'hydroquinone-diallyléther, le tétrallyl-oxéthanoyle ou d'autres allyl ou vinyléthers alcools polyfonctionnels, le diacrylate de tétraéthylèneglycol, la triallylamine, le triméthylolpropane-diallyléther, le méthylen-bis-acrylamide ou le divinylbenzène.

Les monomères de réticulation ayant au moins deux doubles liaisons oléfiniques sont plus particulièrement choisis parmi ceux répondant à la formule générale (IV) suivante : 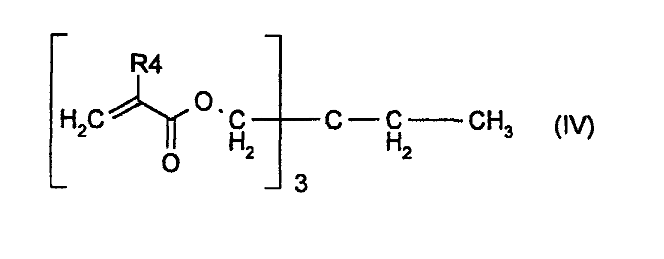 dans laquelle R₄ désigne un atome d'hydrogène ou un radical alkyle en C₁-C₄ et plus particulièrement le radical méthyle (triméthylol propane triacrylate).

Les polymères particulièrement préférés sont ceux présentant une viscosité mesurée au viscosimètre BROOKFIELD, mobile 4, à une vitesse de rotation de 100 tours/minutes, à 25°C, et en solution aqueuse à 2 % en poids, supérieure ou égale à 1000 cps (1 Pa.s) et plus préférentiellement allant de 5000 cps à 40000 cps (5 à 40 Pa.s) et plus particulièrement de 6500 cps à 35000 cps (6,5 Pa.s à 35 Pa.s).

On utilise avantageusement le produit vendu sous le nom de Hostacerin AMPS par la société Hoechst (nom C.T.F.A. : ammonium polyacryldimethyltauramide).

Le gélifiant hydrophile peut également être choisi parmi les copolymères constitués d'une fraction majoritaire de monomère acide carboxylique monooléfiniquement insaturé en C₃-C₆ ou de son anhydride, et d'une fraction minoritaire de monomère ester à chaîne grasse d'acide acrylique. Ces copolymères peuvent être éventuellement réticulés.

Ces copolymères peuvent être préparés en polymérisant une quantité prépondérante de monomère carboxylique monooléfiniquement insaturé ou de son anhydride, avec une quantité plus faible de monomère ester acrylique à chaîne grasse. La quantité de monomère carboxylique ou de son anhydride, est de préférence comprise entre 80 et 98% en poids et plus particulièrement entre 90 et 98% en poids ; l'ester acrylique est de préférence présent dans des quantités comprises entre 2 et 20% en poids et plus particulièrement entre 2 et 10% en poids ; les pourcentages sont calculés par rapport au poids des deux monomères.

Les monomères carboxyliques préférentiels sont choisis parmi ceux répondant à la formule (V) :

CH₂=CR₆-COOH (V)

dans laquelle R₅ désigne hydrogène, halogène, hydroxyle, un groupe lactone, un groupe lactame, un groupe cyanogène (-CN), un groupe alkyle monovalent, un groupe aryle, un groupe alkylaryle, un groupe aralkyle ou un groupe cycloaliphatique. Les monomères carboxyliques particulièrement préférés sont choisis parmi l'acide acrylique, l'acide méthacrylique, l'anhydride maléique, et leurs mélanges.

Les monomères esters acryliques à chaîne grasse sont préférentiellement choisis parmi ceux répondant à la formule (VI) :

CH₂=CR₆-COOR₇ (VI)

dans laquelle R₆ est choisi dans le groupe formé par hydrogène, méthyle et éthyle et R₇ est un groupe alkyle en C₈-C₃₀, un groupe oxyalkylène en C₈-C₃₀, un groupe carbonyloxyalkylène en C₈-C₃₀. Les monomères esters particulièrement préférés sont ceux pour lesquels R₆ est hydrogène ou méthyle, et/ou ceux pour lesquels R₇ est un groupe alkyle en C₁₀-C₂₂. On peut notamment citer les acrylates et méthacrylates de décyle, de lauryle, de stéaryle, de béhényle ou de mélissyle.

Certains de ces copolymères sont notamment décrits dans la demande EP-A-0268164 et sont obtenus selon les méthodes de préparation décrites dans ce même document.

On peut citer plus particulièrement les copolymères vendus sous le nom PEMULEN par la Société GOODRICH, et notamment le copolymère acrylate/C₁₀-C₃₀-alkylacrylate tel que le produit PEMULEN TR 2.

On peut, bien évidemment, utiliser un mélange de plusieurs copolymères tels que ci-dessus définis.

Le gélifiant hydrophile susceptible d'être employé peut également être choisi parmi les copolymères anioniques réticulés d'acrylamide et d'acide 2-acrylamido 2-méthylpropane sulfonique.

Ces copolymères peuvent notamment être réticulés par un composé à polyinsaturation oléfinique tel que ceux choisis dans le groupe constitué par le tétraallyloxyéthane, l'allypentaérythritol, le méthylène bis-acrylamide, l'allyle-sucrose et le pentaérythritol. Préférentiellement, on utilise le méthylène bis-acrylamide, partiellement ou totalement neutralisé par un agent de neutralisation tel que la soude, la potasse, l'ammoniaque ou une amine telle que la triéthanolamine.

De préférence, ledit composé à polyinsaturation oléfinique est présent dans le copolymère à une concentration comprise entre 0,06 et 1 millimole par mole du mélange de monomères.

Les copolymères préférés sont obtenus par copolymérisation, par voie radicalaire, de 15-85% en moles d'acrylamide et de 15-85% en moles d'acide 2-acrylamido 2-méthylpropane sulfonique, notamment de 30-70% en moles d'acrylamide et de 30-70% en moles d'acide 2-acrylamido 2-méthylpropane sulfonique, et encore mieux de 55-70% en moles d'acrylamide et de 30-45% en moles d'acide 2-acrylamido 2-méthylpropane sulfonique.

Par ailleurs, l'acide 2-acrylamido 2-méthylpropane sulfonique peut être généralement au moins partiellement neutralisé sous la forme d'un sel, par exemple par de la soude, par de la potasse, ou par une amine à faible poids moléculaire telle que la triéthanolamine, ou leurs mélanges.

Un copolymère anionique réticulé particulièrement préféré dans le cadre de la mise en oeuvre de la présente invention est notamment disponible sous la dénomination Sepigel 305 vendu par Seppic (nom C.T.F.A. : polyacrylamide/C13-14 isoparaffin/Laureth 7). On peut également citer le produit Simulgel 600 (nom C.T.F.A. : acrylamide / sodium acryloyldimethyltaurate copolymer / isohexadecane / polysorbate 80) vendu par Seppic.

Le gélifiant hydrophile peut comprendre un mélange de ces différents composés. Selon un mode préféré de réalisation de l'invention, le gélifiant est choisi parmi les polymères poly(acide 2-acrylamido 2-méthylpropane sulfonique) réticulé et neutralisé à au moins 90%.

Le gélifiant peut être présent dans la composition selon l'invention en une quantité de 0,3-10% en poids, de préférence de 0,5-5% en poids, et plus préférentiellement de 1-4% en poids, par rapport au poids total de la composition.

Par ailleurs, on a constaté de manière totalement surprenante qu'en utilisant ces gélifiants particuliers, il était possible de préparer aussi bien des émulsions eau-dans-huile que des émulsions huile-dans-eau, en employant les mêmes tensioactifs, à savoir les esters de dextrine définis ci-dessus, qui sont connus pour leur aptitude à former uniquement des émulsions eau-dans-huile.

Généralement, le sens de l'émulsion sera fonction du procédé de préparation de l'émulsion.

Ainsi il est possible de préparer une émulsion E/H (eau-dans-huile) stable lorsque l'on introduit la phase aqueuse dans la phase grasse, et ceci quelle que soit la proportion de chacune des phases. Par ailleurs, il est également possible de préparer une émulsion E/H stable lorsque la quantité de phase huileuse est supérieure à environ 20% en poids par rapport au poids total de la composition.

A l'inverse, l'ajout d'une phase grasse dans une phase aqueuse conduira le plus souvent à une émulsion H/E (huile-dans-eau) stable, et ceci plus particulièrement lorsque la quantité d'huile est inférieure à environ 30% en poids par rapport à la composition et/ou lorsque la quantité d'ester de dextrine est inférieure à environ 3% en poids par rapport à la composition.

L'invention est donc particulièrement remarquable dans ce sens qu'elle permet de préparer, avec les mêmes constituants, dans les mêmes proportions, aussi bien des émulsions E/H que H/E.

La composition selon l'invention comprend en outre un milieu cosmétiquement ou pharmaceutiquement acceptable, c'est-à-dire un milieu compatible avec une application sur les matières kératiniques telles que la peau, les ongles, les cheveux, les cils et sourcils, les muqueuses et les semi-muqueuses, et toute autre zone cutanée du corps et du visage.

La composition comprend une phase aqueuse cosmétiquement ou pharmaceutiquement acceptable, qui peut comprendre de l'eau, une eau florale telle que l'eau de bleuet, une eau minérale telle que l'eau de VITTEL, l'eau de LUCAS ou l'eau de LA ROCHE POSAY et/ou une eau thermale.

La composition selon l'invention peut également comprendre une phase grasse, notamment constituée de corps gras liquides à 25°C, tels que des huiles d'origine animale, végétale, minérale ou synthétique.

Lorsque la composition selon l'invention se présente sous la forme d'une émulsion, ladite phase grasse peut comprendre toute huile cosmétiquement acceptable, dans la mesure où ladite huile permet, en mélange avec la phase aqueuse et les éventuels additifs, l'obtention d'une émulsion stable, c'est-à-dire d'une émulsion qui ne casse pas, qui reste sous forme d'une phase unique pendant au moins 24 heures après stockage à 25°C, sans phénomène de crémage ou de relarguage d'huile.

Les huiles susceptibles d'être employées peuvent éventuellement être volatiles à température ambiante (20-25°C). On entend par huile volatile, tout composé susceptible de s'évaporer au contact de la peau. De préférence, on utilise des huiles dont le point éclair est suffisamment élevé pour permettre l'utilisation de ces huiles en formulation, et suffisamment bas pour obtenir l'effet évanescent souhaité. On emploie de préférence des huiles dont le point éclair est de l'ordre de 40-100°C.

Ces composés volatils peuvent être choisis en particulier parmi les huiles hydrocarbonées et/ou les huiles siliconées, cycliques ou linéaires, seules ou en mélange. On peut ainsi citer les huiles siliconées volatiles, telles que :
- les silicones volatiles cycliques ayant de 3 à 8 atomes de silicium et de préférence de 4 à 6. Il s'agit par exemple de la cyclotétradiméthylsiloxane, de la cyclopentadiméthylsiloxane ou de la cyclohexadiméthylsiloxane,
- les cyclocopolymères du type diméthylsiloxane/méthylalkylsiloxane, tels que la SILICONE FZ 3109 vendue par la société UNION CARBIDE, qui est un cyclocopolymère diméthylsiloxane/méthyloctylsiloxane,
- les silicones volatiles linéaires ayant de 2 à 9 atomes de silicium. Il s'agit par exemple de l'hexaméthyldisiloxane ou un PDMS de faible viscosité (1 cSt). On peut encore citer l'hexaméthyldisiloxane et les alkyltrisiloxanes tels que l'hexylheptaméthyltrisiloxane ou l'octylheptaméthyltrisiloxane.
On peut également citer les huiles volatiles hydrocarbonées, telles que les isoparaffines et notamment l'isohexadécane et l'isododécane.

Parmi les huiles non volatiles, on peut citer :
- les polyalkyl(C₁-C₂₀) siloxanes et notamment ceux à groupements terminaux triméthylsilyle, de préférence ceux dont la viscosité est inférieure à 0,06 m²/s parmi lesquels on peut citer les polydiméthylsiloxanes linéaires et les alkylméthylpolysiloxanes tels que la cétyldiméthicone (nom CTFA),
- les silicones modifiées par des groupements aliphatiques et/ou aromatiques, éventuellement fluorés, ou par des groupements fonctionnels tels que des groupements hydroxyles, thiols et/ou amines.
- les huiles de silicone phénylées, notamment celles de formule (VII) : 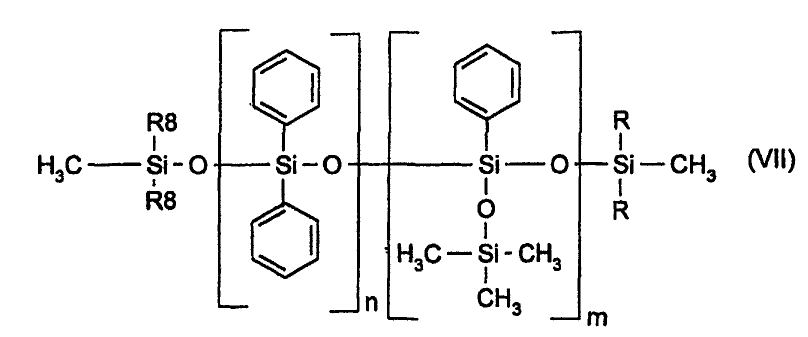
dans laquelle R₈ est un radical alkyle en C1-C30, un radical aryle ou un radical aralkyle, n est un nombre entier compris entre 0 et 100, et m est un nombre entier compris entre 0 et 100, sous réserve que la somme est comprise entre 1 et 100;
- les huiles d'origine animale, végétale ou minérale, telles que l'huile de paraffine, de vaseline, le perhydrosqualène, l'huile d'abricot, l'huile de germes de blé, d'amande douce, de calophyllum, de sésame, de macadamia, de pépins de raisin, de colza, de coprah, d'arachide, de palme, de ricin, d'avocat, de jojoba, d'olive ou de germes de céréales; des esters d'acides gras et de polyol, en particulier les triglycérides liquides; des alcools; des acétylglycérides; des octanoates, décanoates ou ricinoléates d'alcools ou de polyalcools; des triglycérides d'acides gras; des polyalkylènes hydrogénés tels que le polyisobutène (notamment celui commercialisé sous le nom de Parleam par Nippon Oil); des glycérides, les huiles fluorées et perfluorées;
- leurs mélanges.

Lorsque la composition se présente sous forme d'une émulsion huile-dans-eau, la phase grasse de l'émulsion peut être présente à une teneur de 0,5-30% en poids par rapport au poids total de l'émulsion, de préférence de 1-20% en poids.

Lorsque la composition se présente sous forme d'une émulsion eau-dans-huile, la phase grasse de l'émulsion peut être présente à une teneur de 5-98% en poids par rapport au poids total de l'émulsion, de préférence de 10-40% en poids.

La composition selon l'invention peut comprendre en outre d'autres corps gras, qui peuvent être choisis par l'homme du métier sur base de ses connaissances générales, de manière à conférer à la composition finale les propriétés souhaitées, par exemple en consistance, en texture et/ou en transfert. Ces corps gras additionnels peuvent être des cires, des gommes et/ou des corps gras pâteux d'origine animale, végétale, minérale ou synthétique, ainsi que leurs mélanges.
On peut notamment citer :
- les gommes de silicones,
- les cires d'origine animale, végétale, minérale ou synthétique telles que les cires microcristallines, la paraffine, le pétrolatum, la vaseline, l'ozokérite, la cire de montan; la cire d'abeilles, la lanoline et ses dérivés; les cires de Candellila, d'Ouricury, de Camauba, du Japon, le beurre de cacao, les cires de fibres de lièges ou de canne à sucre; les huiles hydrogénées concrètes à 25°C, les ozokérites, les esters gras et les glycérides concrets à 25°C; les cires de polyéthylène et les cires obtenues par synthèse de Fischer-Tropsch; des huiles hydrogénées concrètes à 25°C; des lanolines; des esters gras concrets à 25°C; les cires de silicone; les cires fluorées; leurs mélanges.

La composition selon l'invention peut en outre comprendre un ou plusieurs solvants organiques cosmétiquement acceptables qui peuvent être choisis dans le groupe constitué par les solvants organiques hydrophiles, les solvants organiques lipophiles, les solvants amphiphiles ou leurs mélanges.
Parmi les solvants organiques hydrophiles, on peut citer par exemple des monoalcools inférieurs linéaires ou ramifiés ayant 1 à 8 atomes de carbone comme l'éthanol, le propanol, le butanol, l'isopropanol, l'isobutanol; des polyéthylène glycols ayant de 6 à 80 oxydes d'éthylène; des polyols tels que le propylène glycol, l'isoprène glycol, le butylène glycol, le glycérol, le sorbitol; les mono- ou di-alkyle d'isosorbide dont les groupements alkyle ont de 1 à 5 atomes de carbone; les éthers de glycol comme le diéthylène glycol mono-méthyl ou mono-éthyl éther et les éthers de propylène glycol comme le dipropylène glycol méthyl éther.
Comme solvants organiques amphiphiles, on peut citer des polyols tels que des dérivés de polypropylène glycol (PPG) et notamment les esters de polypropylène glycol et d'acide gras, de PPG et d'alcool gras comme le PPG-23 oléyl éther et le PPG-36 oléate. Comme solvants organiques lipophiles, on peut citer les esters gras tels que l'adipate de diisopropyle, l'adipate de dioctyle, les benzoates d'alkyle.

La composition selon l'invention peut comprendre en outre une phase particulaire qui peut comprendre des pigments et/ou des nacres et/ou des charges habituellement utilisés dans les compositions cosmétiques. Par pigments, il faut comprendre des particules blanches ou colorées, minérales ou organiques, insolubles dans le milieu, destinées à colorer et/ou opacifier la composition. Par charges, il faut comprendre des particules incolores ou blanches, minérales ou de synthèse, lamellaires ou non lamellaires, destinées à donner du corps ou de la rigidité à la composition, et/ou de la douceur, de la matité et de l'uniformité au maquillage. Par nacres, il faut comprendre des particules irisées qui réfléchissent la lumière.

Les pigments peuvent être présents à raison de 0-20 % en poids, par rapport au poids total de la composition, et de préférence à raison de 2-15 %. Ils peuvent être blancs ou colorés, minéraux et/ou organiques, de taille usuelle ou nanométrique. On peut citer, parmi les pigments et les nanopigments minéraux, les dioxydes de titane, de zirconium ou de cérium, ainsi que les oxydes de zinc, de fer ou de chrome, les nanotitanes, le bleu ferrique. Parmi les pigments organiques, on peut citer le noir de carbone, et les laques couramment employées pour conférer aux lèvres et à la peau un effet de maquillage, qui sont des sels de calcium, de baryum, d'aluminium ou de zirconium, de colorants acides tels que les colorants halogéno-acides, azoïques ou anthraquinoniques. Les nacres peuvent être présentes dans la composition à raison de 0-20% en poids, de préférence à un taux élevé de l'ordre de 2-15% en poids. Parmi les nacres envisageables, on peut citer la nacre naturelle, le mica recouvert d'oxyde de titane, d'oxyde de fer, de pigment naturel ou d'oxychlorure de bismuth ainsi que le mica titane coloré.

Les charges, qui peuvent être présentes dans la composition à raison de 0-20 % en poids, par rapport au poids total de la composition, de préférence 2-10%, peuvent être minérales ou de synthèse, lamellaires ou non lamellaires. On peut citer le talc, le mica, la silice, le kaolin, les poudres de Nylon et de polyéthylène, le Téflon, l'amidon, le nitrure de bore, les microsphères telles que l'Expancel (Nobel Industrie), le polytrap (Dow Corning) et les microbilles de résine de silicone (Tospearls de Toshiba, par exemple).

La composition peut également comprendre des colorants hydrosolubles choisis parmi les colorants usuels du domaine considéré tels que le sel disodique de ponceau, le sel disodique du vert d'alizarine, le jaune de quinoléine, le sel trisodique d'amarante, le sel disodique de tartrazine, le sel monosodique de rhodamine, le sel disodique de fuchsine, la xanthophylle.

La composition peut comprendre en outre tout additif usuellement utilisé dans le domaine cosmétique, tel que des antioxydants, des parfums, des huiles essentielles, des conservateurs, des actifs cosmétiques ou pharmaceutiques lipophiles ou hydrophiles, des hydratants, des vitamines, des acides gras essentiels, des sphingolipides, des composés auto-bronzants tels que la DHA, des filtres solaires. Bien entendu l'homme du métier veillera à choisir ce ou ces éventuels composés complémentaires, et/ou leur quantité, de manière telle que les propriétés avantageuses de la composition selon l'invention ne soient pas,
ou substantiellement pas, altérées par l'adjonction envisagée.

Les compositions selon l'invention, qui se présentent donc sous la forme d'une émulsion huile-dans-eau, d'une émulsion eau-dans-huile ou d'une émulsion multiple, trouvent une application notamment dans le domaine du soin, du nettoyage, du démaquillage ou du maquillage de la peau, des semi-muqueuses, des muqueuses et/ou des phanères.

Elles peuvent se présenter par exemple sous la forme d'une composition de maquillage telle qu'un fond de teint, un fard à joues ou à paupières, un rouge à lèvres, un mascara, un eye-liner; d'une composition de soin telle qu'une base de soin pour les lèvres, une crème de soin (crème de jour, de nuit, anti-rides, hydratante), une crème ou émulsion démaquillante ; d'une composition solaire ou autobronzante ; d'une composition capillaire telle qu'une crème de soin des cheveux, cils et sourcils.

L'invention est illustrée plus en détail dans les exemples suivants.

### Exemple 1 : crème nourrissante pour peaux très sèches - émulsion E/H

On prépare une émulsion E/H comprenant :

### phase aqueuse :

- ammonium polyacryldimethyl tauramide (Hostacerin AMPS) 1,5 g
- glycérol 5 g
- conservateurs qs
- eau qsp 100 g

### phase grasse :

- iso-paraffine hydrogénée 27 g
- palmitate de dextrine (Rheopearl KE) 3 g

On disperse la phase aqueuse, préalablement chauffée à 70-75°C, dans la phase grasse préalablement chauffée à 70-75°C, sous agitation.
On obtient une émulsion E/H très riche, nourrissante, apaisante, idéale pour le soin des peaux sèches et/ou irritées.
Cette crème n'est pas grasse ni collante grâce à la présence du gélifiant hydrophile qui apporte un effet frais très agréable. Elle pénètre rapidement dans la peau qui reste douce et souple.
Son aspect au microscope montre une émulsion fine, régulière qui confirme sa bonne stabilité (2 mois à température ambiante et 2 mois à 37°C).

### Exemple 2 : crème nourrissante pour peaux très sèches - émulsion HIE

On prépare une émulsion H/E comprenant :

### phase aqueuse :

- ammonium polyacryldimethyl tauramide 1,5 g
- glycérol 5 g
- conservateurs qs
- eau qsp 100 g

### phase grasse :

- isoparaffine hydrogénée 27 g
- palmitate de dextrine (Rheopearl TL) 3 g

On disperse la phase grasse, préalablement chauffée à 70-75°C, dans la phase aqueuse préalablement chauffée à 70-75°C, sous agitation.
On obtient une émulsion H/E légère et fraîche qui possède de très bonnes qualités de nutrition et un effet apaisant immédiat.
Sa texture est crémeuse, onctueuse, facile à appliquer. Elle pénètre rapidement dans la peau qui reste douce et souple.
Son aspect au microscope montre une émulsion fine, régulière qui confirme sa bonne stabilité (2 mois à température ambiante et 2 mois à 37°C).

### Exemple 3 : comparatif

On prépare une émulsion comparative 1, de type E/H comprenant

### phase aqueuse :

- glycérol 5 g
- conservateurs qs
- eau qsp 100 g

### phase grasse :

- isoparaffine hydrogénée 27 g
- palmitate de dextrine (Rheopearl TL) 3 g
On disperse la phase aqueuse, préalablement chauffée à 70-75°C, dans la phase grasse préalablement chauffée à 70-75°C, sous agitation.

On observe les résultats suivants :

| Emulsions | Observations |
|---|---|
| Exemple 1 | émulsion E/H très riche, nourrissante, apaisante, idéale pour le soin des peaux sèches et/ou irritées, ni grasse, ni collante. Emulsion fine et régulière; stable 2 mois à température ambiante ou 2 mois à 37°C |
| Emulsion comparative 1 (sans gélifiant hydrophile) | émulsion E/H ayant un toucher gras, lourd, collant et une mauvaise stabilité |

### Exemple 4 : crème légère hydratante - émulsion H/E

On prépare une émulsion H/E comprenant :

### phase aqueuse :

- ammonium polyacryldimethyl tauramide 1 g
- glycérol 5 g
- conservateurs qs
- eau qsp 100 g

### phase grasse :

- huile d'amandes d'abricot 10 g
- cyclohexadiméthylsiloxane 10 g
- palmitate de dextrine (Rheopearl KE) 2 g

On disperse la phase grasse, préalablement chauffée à 70-75°C, dans la phase aqueuse préalablement chauffée à 70-75°C, sous agitation. On obtient une émulsion H/E parfaitement stable. Elle est douce et légère. Sa texture est crémeuse, onctueuse.

Si on supprime le palmitate de dextrine, on obtient une émulsion dont la stabilité est nettement inférieure ainsi que les propriétés cosmétiques: texture gélifiée (non crémeuse), moins douce, moins confortable; la prise sur le doigt est moins bonne.

### Exemple 5 : émulsions démaquillantes pour peaux sèches - H/E et E/H

A/ On prépare une émulsion H/E comprenant :

### phase aqueuse :

- ammonium polyacryldimethyl tauramide 1 g
- glycérol 5 g
- conservateurs qs
- eau qsp 100 g

### phase grasse :

- palmitate d'éthyl-2-hexyle 20 g
- cyclopentadiméthylsiloxane 10 g
- palmitate de dextrine (Rheopearl KE) 2 g

On disperse la phase grasse, préalablement chauffée à 70-75°C, dans la phase aqueuse préalablement chauffée à 70-75°C, sous agitation. On obtient une émulsion H/E pour le démaquillage de tous les types de peaux même très sèches et sensibles. Cette émulsion démaquillante est facile à éliminer par simple rinçage avec de l'eau ou un tonique.

B/ On prépare une émulsion E/H comprenant les mêmes constituants que ci-dessus, en dispersant la phase aqueuse dans la phase grasse.
On obtient une émulsion E/H particulièrement adaptée au démaquillage des produits de maquillage longue tenue ou waterproof, grâce à l'action solvante des huiles présentes dans la phase externe.

### Exemple 6 : émulsions E/H et HIE

On prépare deux phases aqueuses et deux phases grasses comprenant :

### phase aqueuse :

- copolymère acrylate/C₁₀-C₃₀-alkylacrylate (PEMULEN TR 2) 0,2%
- NaOH 0,08%
- glycérol 5 %
- eau qsp 100%

### phase grasse :

- isoparaffine hydrogénée 27 g
- palmitate de dextrine (Rheopearl TL) 3 g

On disperse une phase aqueuse, préalablement chauffée à 70-75°C, dans une phase grasse préalablement chauffée à 70-75°C, sous agitation. On obtient une émulsion stable E/H.

On disperse l'autre phase grasse préalablement chauffée à 70-75°C, dans l'autre phase aqueuse préalablement chauffée à 70-75°C, sous agitation. On obtient une émulsion stable H/E.

## Revendications

1. Composition notamment cosmétique ou pharmaceutique comprenant, dans un milieu cosmétiquement ou pharmaceutiquement acceptable, au moins un ester de dextrine et d'acide gras, répondant à la formule (I) : 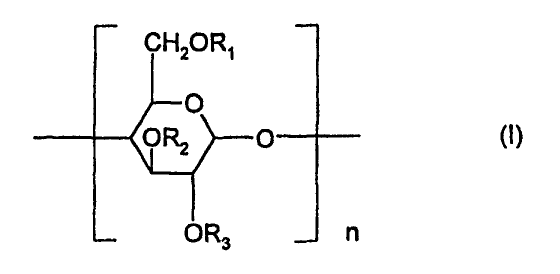 dans laquelle :
- les radicaux R₁, R₂ et R₃, identiques ou différents, sont choisis parmi l'hydrogène ou un groupement acyle (R-CO-) dans lequel le radical R est un groupement hydrocarboné, linéaire ou ramifié, saturé ou insaturé, possédant 6 à 30, notamment 8 à 22, voire 12-18, atomes de carbone, sous réserve qu'au moins un desdits radicaux R₁, R₂ ou R₃ est différent de l'hydrogène,
- n est un entier compris entre 3 et 150, notamment 10 et 100, et de préférence 15-40;
et au moins un gélifiant hydrophile de type polymérique choisi parmi les polymères de type acrylamide ou dérivés, et les copolymères constitués d'une fraction majoritaire de monomère acide carboxylique monooléfiniquement insaturé en C₃-C₆ ou de son anhydride, et d'une fraction minoritaire de monomère ester à chaîne grasse d'acide acrylique.

2. Composition selon la revendication 1, dans laquelle l'ester de dextrine répond à la formule (II) suivante : 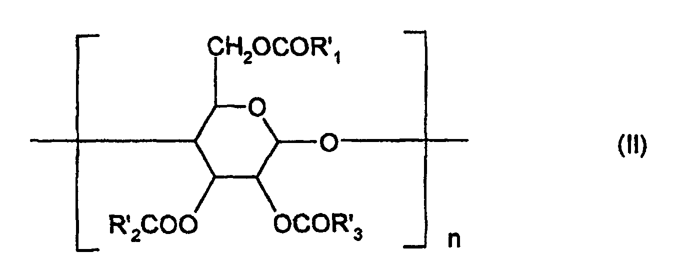 dans laquelle les radicaux R'₁, R'₂ et R'₃, identiques ou différents, représentent un groupement hydrocarboné, linéaire ou ramifié, saturé ou insaturé, possédant 6 à 30, notamment 8 à 22, voire 12-18, atomes de carbone,
- n est un entier compris entre 3 et 150, notamment 10 et 100, et de préférence 15-40.

3. Composition selon la revendication 2, dans laquelle l'ester de dextrine possède des radicaux -OCO R'₁, -OCO R'₂ et/ou -OCO R'₃ choisis parmi les radicaux caprylique, caprique, laurique, myristique, palmitique, stéarique, arachique, behenique, isobutyrique, isovalérique, éthyl-2 butyrique, éthylméthylacétique, isoheptanoïque, éthyl-2 hexanoïque, isononanoïque, isodécanoïque, isotridécanoïque, isomyristique, isopalmitique, isostéarique, isoaracique, isohexanoïque, decenoïque, dodécenoïque, tetradecenoïque, myristoléïque, hexadécénoïque, palmitoléïque, oléïque, élaidique, asclepinique, gondoléïque, eicosènoïque, sorbique, linoléïque, linolénique, punicique, stéaridonique, arachidonique, stéarolique, et leurs mélanges.

4. Composition selon l'une des revendications précédentes, dans laquelle les esters de dextrine sont présents en une quantité de 0,2-10%, de préférence de 0,5-5% en poids, et plus préférentiellement de 1-4% en poids, par rapport au poids total de la composition.

5. Composition selon l'une des revendications précédentes, dans laquelle le gélifiant hydrophile polymérique est un polymère poly(acide 2-acrylamido 2-méthylpropane sulfonique) réticulé et neutralisé à au moins 90%.

6. Composition selon l'une des revendications précédentes, dans laquelle le gélifiant hydrophile polymérique est un polymère poly(acide 2-acrylamido 2-méthylpropane sulfonique) réticulé et neutralisé comprenant, distribués de façon aléatoire :
a) de 90 à 99,9% en poids de motifs de formule générale (III) suivante : 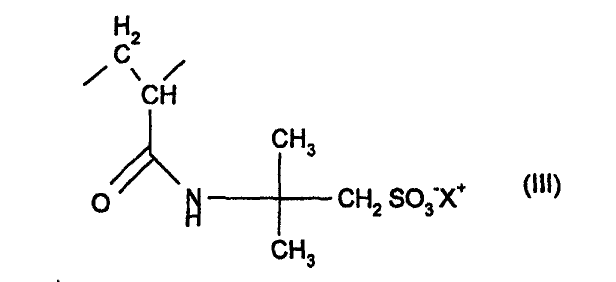 dans laquelle X⁺ désigne un cation ou un mélange de cations, au plus 10% molaire des cations X⁺ pouvant être des protons H⁺ ; et
b) de 0,01 à 10% en poids de motifs réticulants provenant d'au moins un monomère ayant au moins deux doubles liaisons oléfiniques ; les proportions en poids étant définies par rapport au poids total du polymère.

7. Composition selon l'une des revendications 1 à 4, dans laquelle le gélifiant hydrophile polymérique est choisi parmi les copolymères anioniques réticulés d'acrylamide et d'acide 2-acrylamido 2-méthylpropane sulfonique.

8. Composition selon l'une des revendications précédentes, dans laquelle le gélifiant hydrophile polymérique est présent en une quantité de 0,3-10% en poids, de préférence de 0,5-5% en poids, et plus préférentiellement de 1-4% en poids, par rapport au poids total de la composition.

9. Composition selon l'une des revendications précédentes, se présentant sous la forme d'une émulsion E/H (eau-dans-huile) stable ou d'une émulsion H/E (huile-dans-eau) stable, ou d'une émulsion multiple.

10. Composition selon l'une des revendications précédentes, se présentant sous forme d'une émulsion huile-dans-eau et ayant une phase grasse présente à une teneur de 0,5-30% en poids par rapport au poids total de l'émulsion, de préférence de 1-20% en poids.

11. Composition selon l'une des revendications 1-9, se présentant sous forme d'une émulsion eau-dans-huile et ayant une phase grasse présente à une teneur de 5-98% en poids par rapport au poids total de l'émulsion, de préférence de 10-40% en poids.

12. Composition selon l'une des revendications précédentes, se présentant sous la forme d'une composition de soin, de nettoyage, de démaquillage ou de maquillage de la peau, des semi-muqueuses, des muqueuses et/ou des phanères.

13. Composition selon l'une des revendications précédentes, se présentant sous la forme d'une composition de maquillage telle qu'un fond de teint, un fard à joues ou à paupières, un rouge à lèvres, un mascara, un eye-liner; d'une composition de soin telle qu'une base de soin pour les lèvres, une crème de soin (crème de jour, de nuit, anti-rides, hydratante), une crème ou émulsion démaquillante; d'une composition solaire ou autobronzante; d'une composition capillaire telle qu'une crème de soin des cheveux, cils et sourcils.

## Patentansprüche

1. Zusammensetzung und insbesondere kosmetische oder pharmazeutische Zusammensetzung, die in einem kosmetisch oder pharmazeutisch akzeptablen Medium mindestens einen Ester von Dextrin und einer Fettsäure der Formel (I): 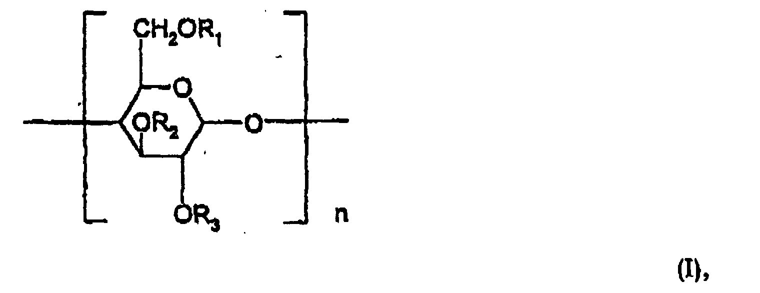 worin:
- die Gruppen R₁, R₂ und R₃, die identisch oder voneinander verschieden sind, unter Wasserstoff oder einer Acylgruppe (R-CO-), worin die Gruppe R eine geradkettige oder verzweigte, gesättigte oder ungesättigte Kohlenwasserstoffgruppe mit 6 bis 30, insbesondere 8 bis 22 oder sogar 12 bis 18 Kohlenstoffatomen bedeutet, ausgewählt sind, mit der Maßgabe, dass mindestens eine der Gruppen R₁, R₂ oder R₃ von Wasserstoff verschieden ist, und
- n eine ganze Zahl von 3 bis 150, insbesondere von 10 bis 100 und vorzugsweise von 15 bis 40 bedeutet, und mindestens einen hydrophilen Gelbildner vom Polymertyp enthält, der unter den Polymeren vom Typ eines Acrylamids oder dessen Derivaten und den Copolymeren, die zum überwiegenden Teil aus Monomeren einer einfach olefinisch ungesättigten Carbonsäure mit 3 bis 6 Kohlenstoffatomen oder des Anhydrids dieser Säure und zu einem kleineren Teil aus Monomeren eines Acrylsäureesters mit Fettkette bestehen, ausgewählt ist.

2. Zusammensetzung nach Anspruch 1, worin der Dextrinester der folgenden Formel (II) entspricht: 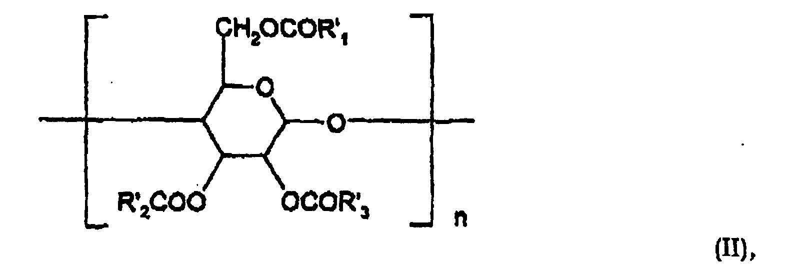 worin bedeuten:
- die Gruppen R'₁, R'₂ und R'₃, die identisch oder voneinander verschieden sind, eine geradkettige oder verzweigte, gesättigte oder ungesättigte Kohlenwasserstoffgruppe mit 6 bis 30, insbesondere 8 bis 22 oder sogar 12 bis 18 Kohlenstoffatomen
und
- n eine ganze Zahl von 3 bis 150, insbesondere von 10 bis 100 und vorzugsweise von 15 bis 40.

3. Zusammensetzung nach Anspruch 2, worin der Dextrinester Gruppen -OCO R'₁, -OCO R'₂, und/oder -OCO R'₃ aufweist, die unter den Resten von Caprylsäure, Caprinsäure, Laurinsäure, Myristinsäure, Palmitinsäure, Stearinsäure, Arachinsäure, Behensäure, Isobuttersäure, Isovaleriansäure, 2-Ethylbuttersäure, Ethylmethylessigsäure, Isoheptansäure, 2-Ethylhexansäure, Isononansäure, Isodecansäure, Isotridecansäure, Isomyristinsäure, Isopalmitinsäure, Isostearinsäure, Isoarachinsäure, Isohexansäure, Decensäure, Dodecensäure, Tetradecensäure, Myristoleinsäure, Hexadecensäure, Palmitoleinsäure, Ölsäure, Elaidinsäure, Asclepininsäure, Gondoleinsäure, Eicosensäure, Sorbinsäure, Linolsäure, Linolensäure, Punicinsäure, Stearidonsäure, Arachidonsäure, Stearoleinsäure und den Gemischen dieser Säurereste ausgewählt sind.

4. Zusammensetzung nach einem der vorhergehenden Ansprüche, worin die Dextrinester in einem Mengenanteil von 0,2 bis 10 Gew.-%, vorzugsweise von 0,5 bis 5 Gew.-% und noch bevorzugter von 1 bis 4 Gew.-%, bezogen auf das Gesamtgewicht der Zusammensetzung, vorliegen.

5. Zusammensetzung nach einem der vorhergehenden Ansprüche, worin der polymere hydrophile Gelbildner ein Poly(2-acrylamido-2-methylpropan-sulfonsäure)-Polymer ist, das vernetzt und zu mindestens 90 % neutralisiert ist.

6. Zusammensetzung nach einem der vorhergehenden Ansprüche, worin der polymere hydrophile Gelbildner ein vernetztes und neutralisiertes Poly(2-acrylamido-2-methylpropan-sulfonsäure)-Polymer ist, das in zufälliger Verteilung enthält:
a) 90 bis 99,9 Gew.-% Einheiten der folgenden allgemeinen Formel (III): 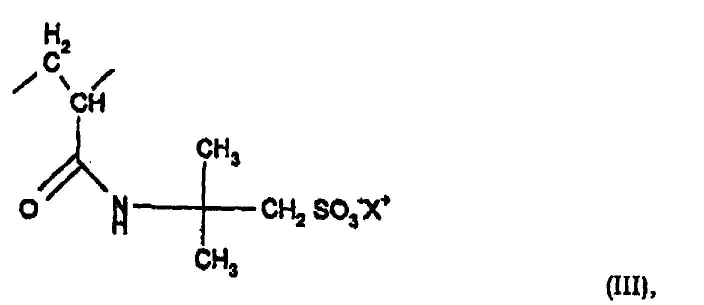 worin X⁺ ein Kation oder ein Gemisch von Kationen bedeutet, wobei höchstens 10 Mol-% der Kationen X⁺ Protonen H⁺ sein können,
und
b) 0,01 bis 10 Gew.-% vernetzende Einheiten, die von mindestens einem Monomer stammen, das mindestens zwei olefinische Doppelbindungen aufweist,
wobei die Gewichtsanteile bezogen auf das Gesamtgewicht des Polymers angegeben sind.

7. Zusammensetzung nach einem der Ansprüche 1 bis 4, worin der polymere hydrophile Gelbildner unter den vernetzten anionischen Copolymeren von Acrylamid und 2-Acrylamido-2-methylpropan-sulfonsäure ausgewählt ist.

8. Zusammensetzung nach einem der vorhergehenden Ansprüche, worin der polymere hydrophile Gelbildner in einem Mengenanteil von 0,3 bis 10 Gew.-%, vorzugsweise von 0,5 bis 5 Gew.-% und noch bevorzugter von 1 bis 4 Gew.-%, bezogen auf das Gesamtgewicht der Zusammensetzung, vorliegt.

9. Zusammensetzung nach einem der vorhergehenden Ansprüche, die in Form einer stabilen W/O-Emulsion (Wasser-in-Öl) oder einer stabilen O/W-Emuls (Öl-in-Wasser) oder einer multiplen Emulsion vorliegt.

10. Zusammensetzung nach einem der vorhergehenden Ansprüche, die in Form einer Öl-in-Wasser-Emulsion vorliegt und eine Fettphase aufweist, die in einem Mengenanteil von 0,5 bis 30 Gew.-% und vorzugsweise von 1 bis 20 Gew.-%, bezogen auf das Gesamtgewicht der Emulsion, vorliegt.

11. Zusammensetzung nach einem der Ansprüche 1 bis 9, die in Form einer Wasser-in-Öl-Emulsion vorliegt und eine Fettphase aufweist, die in einem Mengenanteil von 5 bis 98 Gew.-% und vorzugsweise von 10 bis 40 Gew.-%, bezogen auf das Gesamtgewicht der Emulsion, vorliegt.

12. Zusammensetzung nach einem der vorhergehenden Ansprüche, die in Form einer Zusammensetzung zur Pflege, zum Reinigen, zum Abschminken oder zum Schminken der Haut, der Semischleimhäute, der Schleimhäute und/oder der Hautanhangsgebilde vorliegt.

13. Zusammensetzung nach einem der vorhergehenden Ansprüche, die in Form einer Zusammensetzung zum Schminken, wie als Make-up, Wangenrouge, Lidschatten, Lippenstift, Mascara oder als Eyeliner, einer Zusammensetzung zur Pflege, wie als Pflegegrundmasse für die Lippen, Pflegecreme (Tagescreme, Nachtcreme, Antifalten-Creme, Feuchtigkeitscreme) oder als Creme oder Emulsion zum Abschminken, einer Zusammensetzung zum Sonnenschutz oder zur Selbstbräunung oder einer Zusammensetzung für die Haare, wie als Pflegecreme für die Haare, Wimpern und Augenbrauen, vorliegt.

## Claims

1. Composition, especially a cosmetic or pharmaceutical composition, comprising, in a cosmetically or pharmaceutically acceptable medium, at least one fatty acid ester of dextrin, corresponding to formula (I): 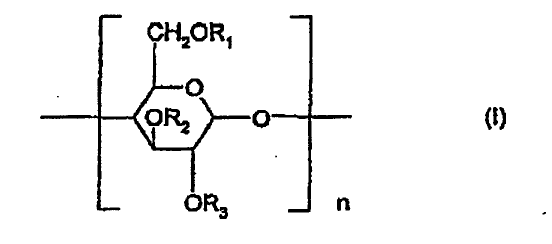 in which:
- the radicals R₁, R₂ and R₃, which may be identical or different, are chosen from hydrogen or an acyl group (R-CO-) in which the radical R is a linear or branched, saturated or unsaturated hydrocarbon-based group containing 6 to 30, especially 8 to 22 or even 12-18 carbon atoms, with the proviso that at least one of the said radicals R₁, R₂ and R₃ is other than hydrogen,
- n is an integer between 3 and 150, especially 10 and 100 and preferably 15-40;
and at least one hydrophilic gelling agent of polymeric type chosen from polymers of acrylamide type or derivatives, and copolymers consisting of a major fraction of C₃-C₆ monoolefinically unsaturated carboxylic acid monomer or the anhydride thereof, and a minor fraction of ester monomer containing an acrylic acid fatty chain.

2. Composition according to Claim 1, in which the dextrin ester corresponds to formula (II) below: 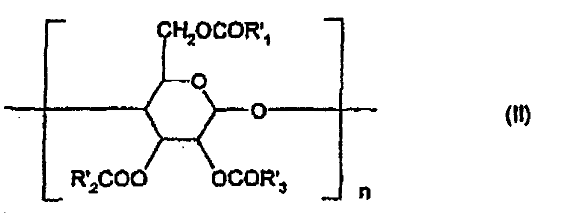 in which the radicals R'₁, R'₂ and R'₃, which may be identical or different, represent a linear or branched, saturated or unsaturated hydrocarbon-based group containing 6 to 30, especially 8 to 22 or even 12-18 carbon atoms,
- n is an integer between 3 and 150, especially 10 and 100 and preferably 15-40.

3. Composition according to Claim 2, in which the dextrin ester contains radicals -OCOR'₁, -OCOR'₂ and/or -OCOR'₃ chosen from caprylic, capric, lauric, myristic, palmitic, stearic, arachic, behenic, isobutyric, isovaleric, 2-ethylbutyric, ethylmethylacetic, isoheptanoic, 2-ethylhexanoic, isononanoic, isodecanoic, isotridecanoic, isomyristic, isopalmitic, isostearic, isoarachic, isohexanoic, decenoic, dodecenoic, tetradecenoic, myristoleic, hexadecenoic, palmitoleic, oleic, elaidic, asclepinic, gondoleic, eicosenoic, sorbic, linoleic, linolenic, punicic, stearidonic, arachidonic and stearolic radicals, and mixtures thereof.

4. Composition according to one of the preceding claims, in which the dextrin esters are present in an amount of 0.2-10%. preferably 0.5-5% by weight and more preferably 1-4% by weight relative to the total weight of the composition.

5. Composition according to one of the preceding claims, in which the polymeric hydrophilic gelling agent is a crosslinked and at least 90% neutralized poly(2-acrylamido-2-methylpropanesulphonic acid) polymer.

6. Composition according to one of the preceding claims, in which the polymeric hydrophilic gelling agent is a crosslinked and neutralized poly(2-acrylamido-2-methylpropanesulphonic acid) polymer comprising, randomly distributed:
a) from 90% to 99.9% by weight of units of general formula (III) below: 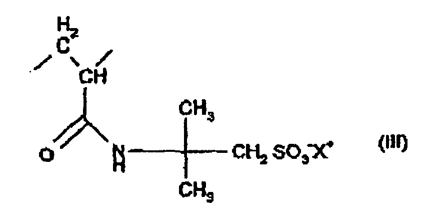 in which X⁺ denotes a cation or a mixture of cations, not more than 10 mol% of the cations X⁺ possibly being protons H⁺; and
b) from 0.01% to 10% by weight of crosslinking units derived from at least one monomer containing at least two olefinic double bonds; the weight proportions being defined relative to the total weight of the polymer.

7. Composition according to one of Claims 1 to 4, in which the polymeric hydrophilic gelling agent is chosen from crosslinked anionic copolymers of acrylamide and of 2-acrylamido-2-methylpropanesulphonic acid.

8. Composition according to one of the preceding claims, in which the polymeric hydrophilic gelling agent is present in an amount of 0.3-10% by weight, preferably 0.5-5% by weight and more preferably 1-4% by weight relative to the total weight of the composition.

9. Composition according to one of the preceding claims, which is in the form of a stable W/O (water-in-oil) emulsion or a stable O/W (oil-in-water) emulsion, or a multiple emulsion.

10. Composition according to one of the preceding claims, which is in the form of an oil-in-water emulsion containing a fatty phase present in a content of 0.5-30% by weight relative to the total weight of the emulsion, and preferably 1-20% by weight.

11. Composition according to one of Claims 1-9, which is in the form of a water-in-oil emulsion containing a fatty phase present in a content of 5-98% by weight relative to the total weight of the emulsion, and preferably 10-40% by weight.

12. Composition according to one of the preceding claims, which is in the form of a care, cleansing, makeup-removing or makeup composition for the skin, semi-mucous membranes, mucous membranes and/or integuments.

13. Composition according to one of the preceding claims, which is in the form of a makeup composition such as a foundation, a face powder, an eyeshadow, a lipstick, a mascara or an eyeliner; a care composition such as a lipcare base, a care cream (day cream, night cream, anti-wrinkle cream or moisturizing cream) or a makeup-removing cream or emulsion; an antisun or self-tanning composition; a haircare composition such as a care cream for the hair, the eyelashes and the eyebrows.
